# EUROPEAN PATENT APPLICATION

(11) **EP 1 723 971 A1**
(43) Date of publication of application: **22.11.2006**
(21) Application number: 05291100.5
(22) Date of filing: 20.05.2005
(51) Int. Cl.: A61K 45/06

(54) **Methods and compositions for the treatment of viral diseases**

(71) Applicant: Immunoclin Limited, London N12 8NP (GB); Institut de Recherche pour le Développement, 75480 Paris Cedex 10 (FR)
(72) Inventor: Veas, Francisco, 240, Av.Emile Jeanbrau 34094 Montpellier (FR); Missé, Dorothée, 240,av. Emile Jeanbrau 34094 Montpellier (FR); Bray, Dorothy, 289-293 Ballards Lane London N12 8NP (GB); Clerici, Mario, Via GB Grassi 74 20157 Milano (IT)
(74) Representative: McQueen, Andrew Peter

(57) **Abstract**

The invention provides a method of treating viral haemorrhagic fevers, such as that caused by Dengue virus, which comprises administering a composition comprising a pharmaceutically active amount of a matrix metalloproteinase inhibitor.

## Description

This invention relates to the treatment of viral diseases, more particularly it relates to the treatment of viral haemorrhagic fever such as that caused by Dengue virus.
Dengue virus (DV) are arthropod-borne belonging to the flaviviridae family that cause serious human diseases all over the world (Mackenzie et al., Nature Medicine 2004). Four serotypes (DV 1-4) share the world of DV (Zulueta et al., BBRC 2003), and infection by each one of them may result in either a relatively benign febrile course called Dengue fever (DF) or fatal outcomes, such as Dengue hemorrhagic fever (DHF) and Dengue Shock syndrome (DSS) (Huan-Yao Lei et al., J Biomed Sci 2001; Geisbert et al., Nature Medicine 2005; Mackenzie et al., Nature Medicine 2005). The severe form of DV infection DHF/DSS is a vascular leak syndrome that is thought to be precipitated by an immunological cascade beginning with infection of cells of the monocytic lineage, which produce cytokines and other chemical mediators, ultimately leading to increased vascular permeability, leakage, hypovolemia, shock and death if not corrected (Yun-Chi Chen et al., J Virol 2002; Geisbert, Nature Medicine 2005). There is no chemotherapy for DV infection. Less commonly DV infection can cause other severe disease manifestations such as massive haemorrhage, organ failure and neurological disease that mimics viral encephalitis (Gubbler., 1998). Nevertheless, although there are many proposed mechanisms, the pathogenesis of DHF and DSS is still far from understood. DV primarily targets immature dendritic cells (DCs) after a bite by an infected mosquito vector. Different reports show that dendritic-cell-specific ICAM3-grabbing non-integrin (DC-SIGN) is essential for the productive infection of human dendritic cell (Navarro-Sanchez et al., 2003 EMBO report; Tassaneetrithep et al., J exp Med 2003). DC patrols almost every place in human body migrating through the extracellular matrix of different tissues. Upon encounter with non self antigens, DCs rapidly migrate to regional lymph nodes, where they activate naïve T cells to start the immune response (Banchereau, Nature 1998). Cytokines play an important role in the pathogenesis of DV infection, and serum levels of certain cytokines are elevated during DV infection. Ho et al., 2001 (J. Immunol 2001) show that the infection of DC by DV induced production of TNF-α and IFN-α TNF-α is a cytokine that has been implicated widely in conditions associated with vascular leakage, including haemorrhagic fever (Green et al., 1999; J. Infection Disease). Inflammatory cytokines, such as TNF-α and IL-1, as well as bacterial products and viruses, are known to stimulate the maturation and migration of DCs from resident tissues to the lymph nodes.

The prior art is lacking effective treatments for viral haemorrhagic fevers such as that caused by Dengue virus. It is an object of the present invention to provide new and effective methods and compositions for the treatment of these diseases.

Accordingly, the invention provides a method of treating viral haemorrhagic fever, which comprises administering a composition comprising a pharmacologically active amount of a matrix metalloproteinase inhibitor to a patient in need thereof.
Although the invention will be described with reference to its preferred embodiment of a treatment for haemorrhagic fever caused by Dengue virus infection, it will be understood by those skilled in the art that the method and compositions of the invention can be applied to the treatment of any viral haemorrhagic fever. Some types of haemorrhagic fever are tick-borne, others mosquito-borne, and some seem to be zoonoses ; clinical manifestations are high fever, scattered petechiae, gastrointestinal tract and other organ bleeding, hypotension, and shock ; kidney damage may be severe, and neurologic signs may appear.
In particular, the method and compositions of the invention may be applied to haemorrhagic fever caused by infections by viruses of the families Arenaviridae (Lassa fever, Bolivian haemorrhagic fever, Argentinean haemorrhagic fever), Bunyaviridae (Crimean-Congo haemorrhagic fever, haemorrhagic fever with renal syndrome), Flaviviridae (Dengue haemorrhagic fever, Omsk haemorrhagic fever), Filoviridae (Ebola fever, Marburg virus disease), etc.

In a particularly preferred embodiment the haemorrhagic fever is caused by Dengue virus.

Immune cells, including DCs, migrate through the tissues, a process that requires degradation of the extracellular matrix. The invention arises from the inventors' finding that matrix-degrading proteinases are secreted by dengue-infected DCs and are involved in the structure loss of the endothelial cell junctions that could explain the pathogenesis observed during some Dengue infection stages.

In the current study, the inventors show that dengue virus-infected iDCs supernatants upregulate the MMP-2, MMP-9 and MMP-13 gelatinases expression contributing to the increase of permeability of cultured primary human umbilical vein endothelial cells (HUVEC). This permeability was inhibited by both SB-3CT, a potent MMP inhibitor (Kleifeld et al., 2000 JBC 2001) and also by an upstream MMP inhibitor SB203580, a potent inhibitor of the MAPkinase p38. Additionally, the inventors' data also show that the MMP-dependent inhibition of permeabilization was strongly associated with the maintenance of the junctional adhesion proteins, platelet-endothelial cell adhesion molecule-1 (PECAM-1), VE-cadherin, integrin, and keratine and F-actin stress fibres.

Accordingly, in a preferred embodiment, the MMP inhibitor is an inhibitor of gelatinase or collagenase. For example, the inhibitor is an MMP-2, MMP-9 or MMP-13 inhibitor. Most preferably, the inhibitor is an MMP-9 inhibitor.

The data disclosed herein show that the increased vascular permeability associated with infection by Dengue virus can be inhibited by specific inhibitors of MMPs such as the synthetic competitive inhibitor SB-3CT (Kleifeld et al., 2000 JBC 2001), which binds to the catalytic zinc ion of MMP and provides a potent and highly selective inhibition of human gelatinase. Any inhibitor having the required properties of potent and selective inhibition of MMPs could equally be used.

In addition, the inventors' data show that Dengue virus induced secretion of MMPs by iDCs involves activation of the MAPK pathway. Accordingly, molecules which interfere with the MAPK signalling pathway, for example, inhibitors of p38, can be used to inhibit virus-induced MMP production and, hence, prevent the increased vascular permeability associated with the disease. A suitably specific inhibitor of p38 MAPK is SB203580.

Accordingly, suitable MMP inhibitors for use in the method of the invention include at least the following families and or derivatives thereof:
- (i): MAP Kinase inhibitors type SB 203580 : 4-(4-Fluorophenyl)-2-(4-methylsulfinylphenyl)-5-(4-pyridyl)1H-imidazole ;
- (ii): *N*-[(2*R*)-2-(hydroxaminocarbonylmethyl)-4-methylpantanoyl]-L-tryptophan methylamide, GM6001, CA ;
- (iii): SB-3TC: 3-(4-phenoxyphenyl sulfonyl)-propylthiirane.

Derivatives of the foregoing compounds may be produced by routine methods and screened for improved activity, selectivity and reduced toxicity as well as other pharmacologically desirable characteristics. As used herein, a derivative of a compound is a chemically modified version of that compound e.g. with one or more different side groups, or the same reactive groups but modified structure, said modifications modulating the characteristics of the compound, but being sufficiently structurally similar to the original compound to retain MMP inhibitory function. Similarly, other classes of compounds may be screened tor MMP inhibitory activity, low toxicity etc.

In addition to the treatment of Dengue virus infection, the present invention is applicable in the treatment of infections caused by any haemorrhagic fever virus (Marbourgh, Junin, Lassa, Ebola, Hanta etc.) because infections caused by some of these viruses show markers consistent with the endothelial disruption mechanism described herein.

Embodiments of the present invention will now be described purely by way of non-limiting example in which reference is made to the figures of which :

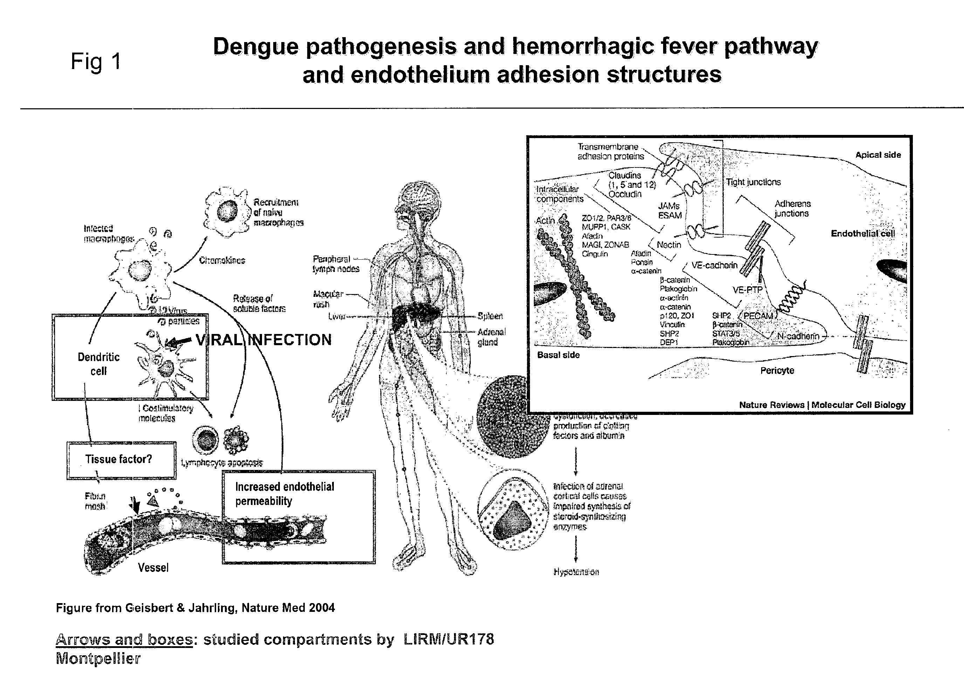

Fig 1. Pathogensis pathways of DEN-V and endothelium junctions details.

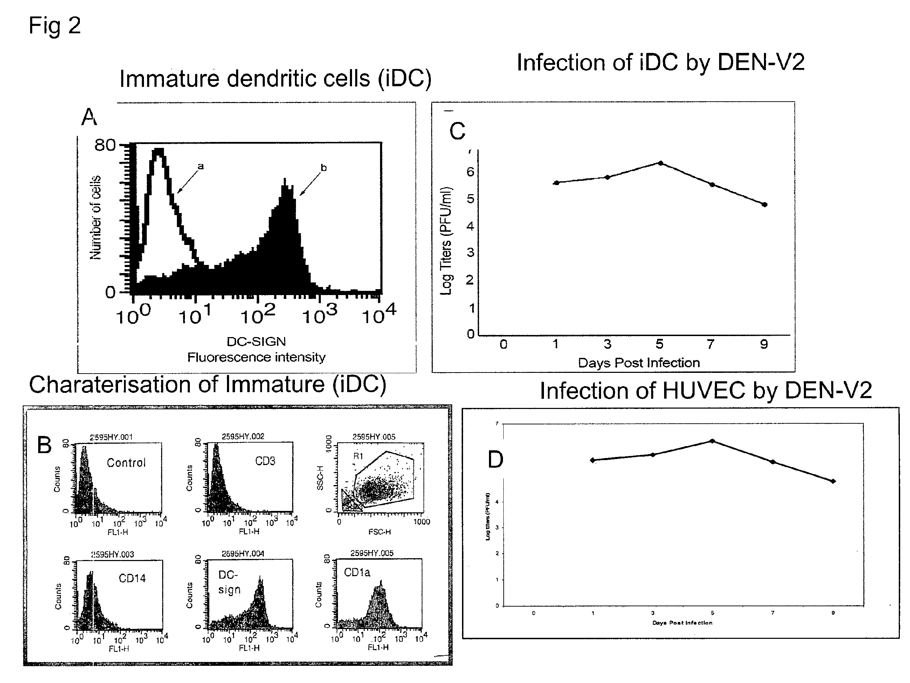

Fig 2. In vitro primary of monocytes-derived iDC infected by DEN-V2. Generation and identification of DC from human peripheral blood. Whole blood was obtained from healthy donors. After mixing with Ficoll-Hypaque and centrifugation, the layer containing mononuclear cells was collected. The adherent mononuclear cells were cultured and maintained in complete medium containing IL-4 and GM-CSF. After culturing for 7 days, (A) the detached cells were stained with DC-SIGN (b) or isotype-matched control mAb (a) ; (B) to control iDC stage CD1 a mAB was also used (C) Immature iDC or (D) HUVEC were infected for 2 hours with DV at an MOI 1 and supernatants of DEN-V-infected iDC were collected from day 1 up to day 9 post infection to titrate DEN-V2 content on LLCK cell line. Data shown are representative of at least 5 independent experiments.

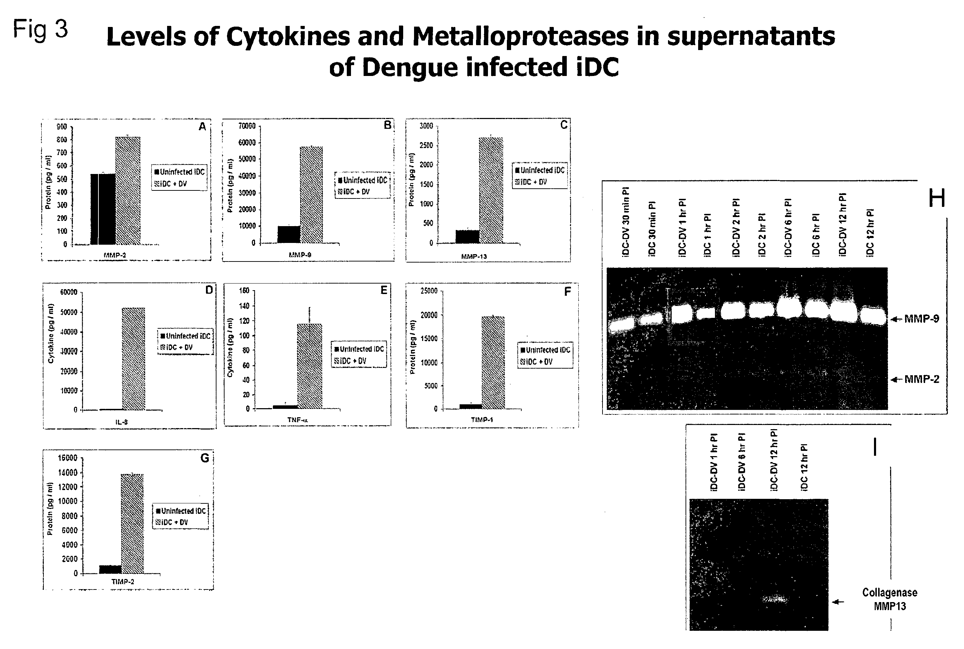

Fig 3. Levels of Metalloproteases Cytokines and natural MMP inhibitors TIMP(1 and 2) in virologically neutralized (by formalin) supernatants of DEN-V infected iDC. iDC were infected with 1 MOI of DEN-V2 for 2 hr supernatants were collected at different time periods, lyophilized, and assayed for their Cytokines, TIMP1 or MMP content (A-G). For MMP2 and MMP9 gelatinase content by zymography and for MMP-13 collagenase content by casein zymography. Data are representative ot 3 or 4 independent experiments (H and I).

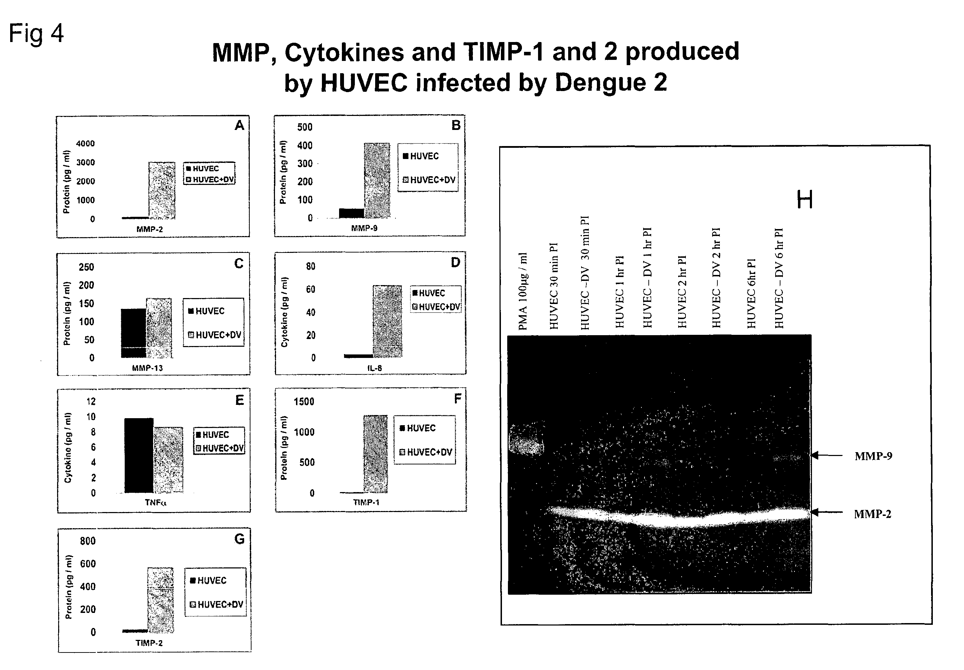

Fig 4. Levels of Metalloproteases, Cytokines and TIMP1 and 2, Cytokines in virologically neutralized (by formalin) supernatants of DEN-V infected HUVEC. HUVEC were infected with 1 MOI of DEN-V2 for 2 hr supernatants were collected at different time periods, lyophilized, and assayed for their Cytokines, TIMP1 or MMP content. For MMP2 and MMP9 gelatinase content by zymography. Data are representative of 3 or 4 independent experiments.

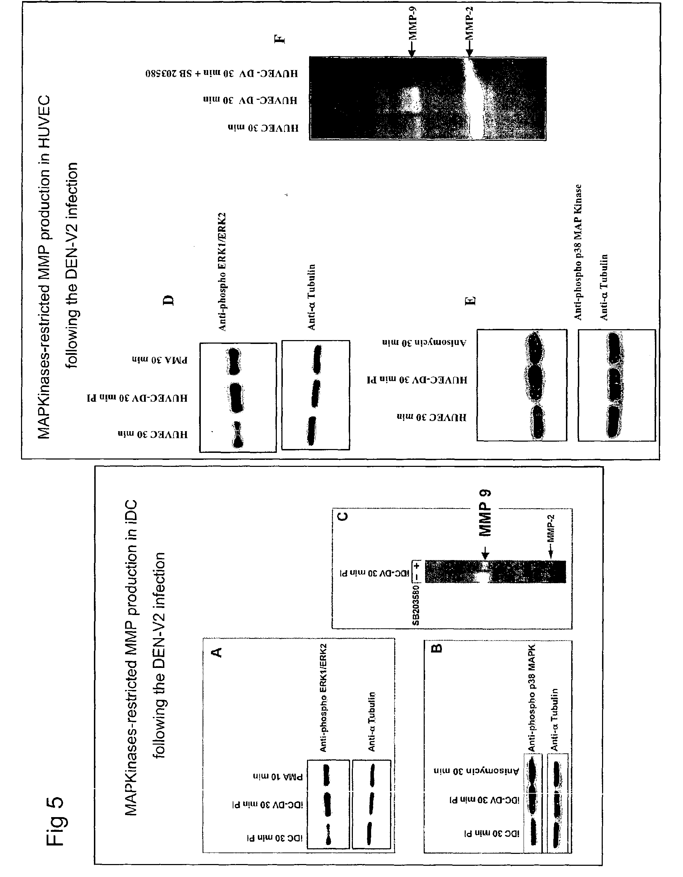

Fig 5. Immature DC (A-C) or HUVEC (D-F) were infected or not with DV and cells were collected 30 min post infection. Cells lysate were separated on 12.5% SDS-PAGE under reducing conditions. Proteins were electrophoretically transferred onto nitrocellulose membrane. The membrane was probed with (A and E) antibodies to total and to specific phosphorylated forms of ERK1/ERK2 antibodies to total and (B, D) to specific phosphorylated forms of p38 kinase. Horseradish peroxidase-conjugated goat anti-rabbit IgG was used as secondary antibody. Immature DC (C) or HUVEC (F) were pretreated or not with 10 µM SB 203580 prior to exposure with DV for 16 hr at 37°C. Supernatants were collected, lyophilized and assayed by zymography. Data are representative of 3 independent experiments.

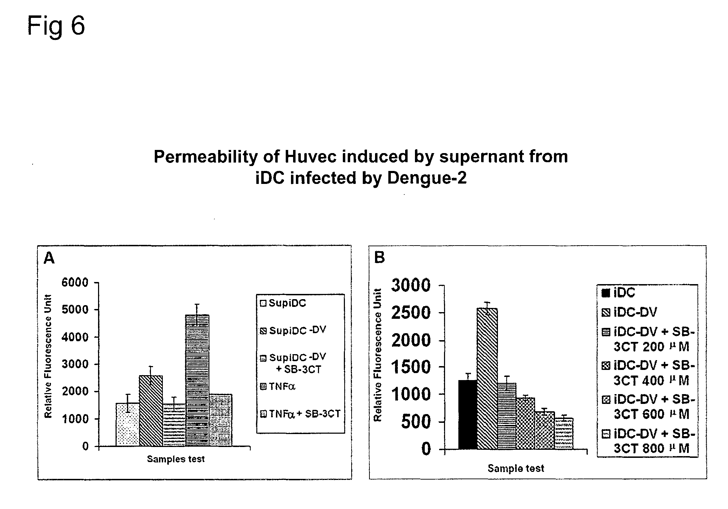

Fig. 6. Virologically neutralized (by formalin) supernatant of DEN-V2 infected-iDC increase endothelial cell permeability in a MMP dependent fashion. Eighty percent confluent HUVEC were prepared on 24-well transwell polycarbonate. (A) cells were pretreated or not with 200 µM SB-3CT for 24 hours before exposure for 12 hours with either culture supernatant of uninfected, DV-infected iDC or recombinant TNFα (20 ng/ml). HUVEC monolayer permeability was measured by using the FITC-Dextran detected with spectrofluorometer at an excitation wavelength of 485 nm and emission at 530 nm. (B) SB-3CT blocks endothelial cell permeability in a dose-dependent manner. Data are representative of 3 or 4 independent experiments.

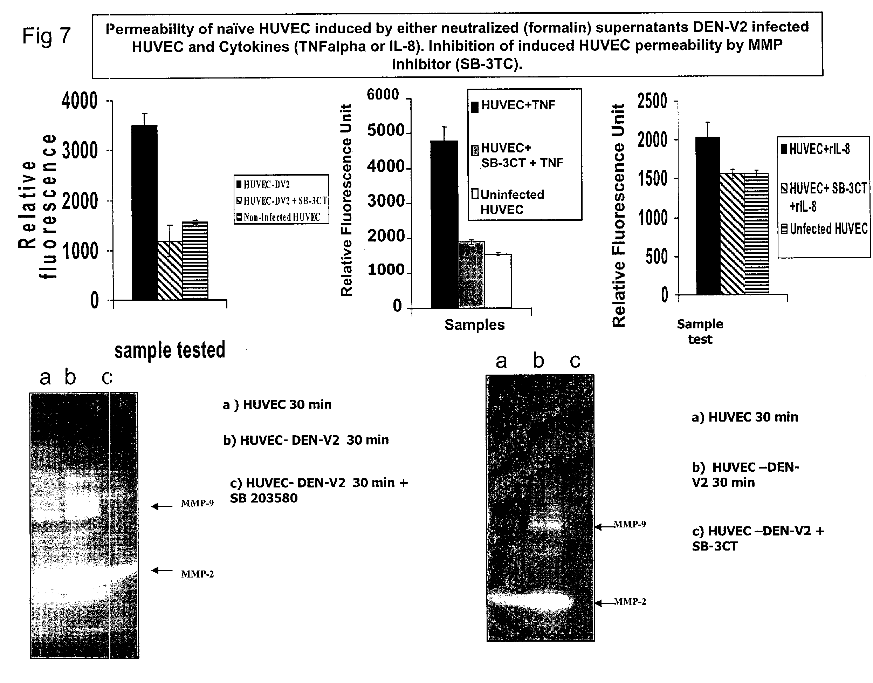

Fig. 7. Virologically neutralized (by formalin) supernatant of DEN-V2 infected-HUVEC increase endothelial cell permeability in a MMP dependent fashion. IL8 or TNF were used as controls. The induced permeability was inhibited as in experiment of Fig 6 by SB-3CT.

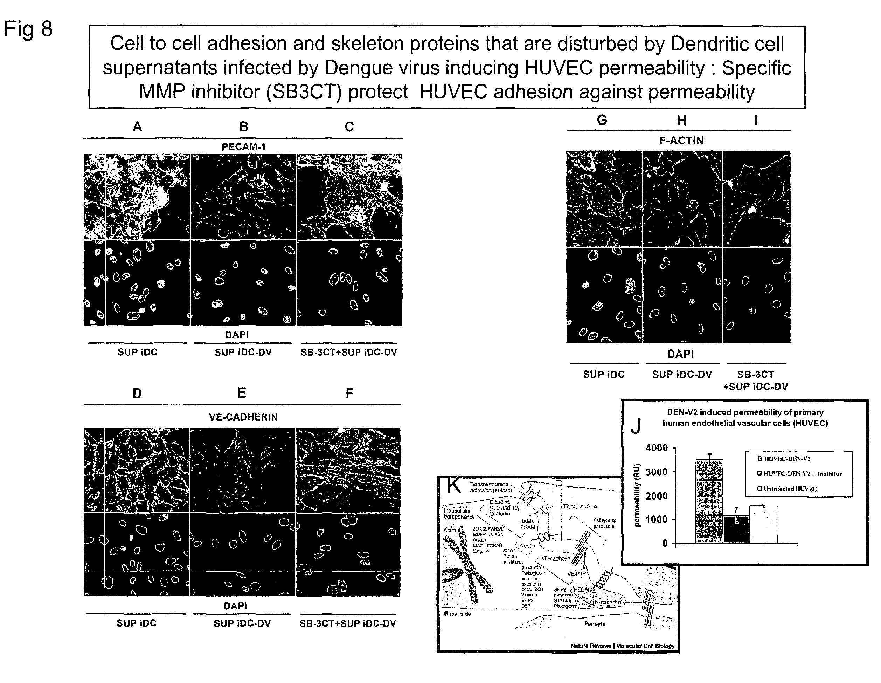

Fig. 8. DV infection of iDC induces MMP-mediated disruption of endothelial cell-cell adhesion. Double immunofluorescence microscopy was carried out on confluent HUVEC monolayers following 12 hours exposure with either culture supernatant of iDC or supernatant of DV-infected iDV. In some experiments, cells were pretreated 200 µM SB-3CT prior to exposure with the culture supernatant. The cells were stained (upper panels) with either PECAM-1 (green : A,B,C), VE-cadherin (D,E,F) or phalloidin (red, G,H,I) for visualizing F-actin filaments. Nuclei were visualized (lower panels) by DAPI staining (purple). Permeability of HUVEC decrease up to the basal level when the SB 3TC MMP-inhibitor is added (J) illustrating the microscopical observations (A-I) on different cell adhesion proteins (K).

### Example

### Summary

Dengue virus (DV) is a human pathogen that causes large epidemics and tens of thousands of deaths annually in many parts of the intertropical world. Despite one of the critical pathogenic effects of Dengue is the hemorrhagic syndrome, many of these aspects this pathogenesis remain largely unclear. The syndrome of "viral hemorrhagic fever" is a consequence shared by other virus infection, such as Ebola, Lassa, and Crimean-congo often associated with a shock syndrome of undetermined pathogenesis. The vascular endothelium seems to be directly and indirectly targeted by all these viruses. Recently, several studies demonstrated that the natural hosts for DV are immature dendritic cells (iDC). These cells were permissive for DV by binding its envelope proteins to DC-SIGN receptor. In this study, we show that iDC and endothelial cells exposed to dengue virus produce a high level of matrix metalloproteinases (MMPs), MMP-9, MMP-2 or MMP-13. These observations indicated that iDC after infected with dengue virus up-regulate the expression of these MMP contributing to a dramatically degradation of the extracellular matrix, destroying the vascular endothelium integrity, and consequently a high increased permeability which is the hallmark of the pathogenesis of DHF/DSS. Interestingly, this increase in permeability can be inhibited by both a specific inhibitor of Mitogen Activated Protein Kinase (MAPK) p38 (for example SB203580) and specific an MMP inhibitors (for example SB3CT). These inhibitors allow the maintain of cell-cell junction structures such as the adhesion proteins, PECAM-1 and VE-cadherin, and the integrity of the content of F-actin stress fibers. Altogether our results provide potential mechanism of the pathogenesis of DHF, such as MMP-production pathway as a critical therapeutic target to overcome cytopathogenic effect not only during Dengue infection and but also during the other viral hemorrhagic fever infections.

### Materials and Methods

### Cells and Virus

iDC were generated from human PBMC by Ficoll-Hypaque density gradient centrifugation. The isolated cells were cultured in 2% gelatin coated dish, maintained with complete IMDM media (Life Technologies, Cergy Pontoise, France) in the presence of granulocyte-macrophage colony-stimulating factor (GM-CSF, 100 ng/ml), and Interleukin 4 (IL-4, 10 ng/ml) purchased at R&D Systems (UK). Five days later all non-adherent cells were harvested, and replated in fresh serum free RPMI 1640 media (Life Technologies, Cergy Pontoise, France). Primary human endothelial cells (ECs) from umbilical vein (HUVECs) were cultured in MCDB 131 medium (Life Technologies, Cergy Pontoise, France Gibco-BRL) supplemented with 2 mM Glutamax (Life Technologies, Cergy Pontoise, France), 10% fetal calf serum (FCS), 10 U/ml porcine heparin (Sigma, Cedex, France), 10 ng/ml hu-EGF (Peprotech, Inc. USA), 35 µg/ml endothelial cell growth supplement (ECGS, BD, Biosciences) and 1 µg/ml hydrocortisone (Sigma, Cedex, France). Experiments were carried out with cells at a 80% of confluence. LLC-MK2 cell line were obtained from the American Type Culture collection (ATCC; Rockville, MD) and were grown in complete Medium 199 (Life Technologies, Cergy Pontoise, France), supplemented with 10% heat-inactivated (56°C for 30 min) fetal bovine serum (Life Technologies, Cergy Pontoise, France), 2 mM glutamax, 1.25 g/L of sodium bicarbonate, 100 units/ml of penicillin G, and 100 µg/ml of streptomycin. The parental DV2- 16681 virus strain, was grown, propagated and titrated by plaque assay in the LLC-MK2 mammalian cell line (Huang et al., J Virol 2003).

### Infection of Dengue-2-16681 to iDC

Immature dendritic cells 5x105 cells/well were exposed to DV2-16681 for 2 hr, at MOI of 1 pfu/cell, in serum free RPMI 1640. Five extensive washes were done to eliminate free virus. Viral replication was followed-up from the day 1 up to the day 9 post infection. Supernatants of DV infected iDC were collected and assessed for their viral activity. For soluble proteins detection, supernatant and cells were collected at different time points (0.5, 1, 2, 6, 12 hours post infection).

### Antibodies and Reagents

Rabbit antihuman antibodies to total and to specific phosphorylated forms of p38 kinase and ERK/MAPK were purchased from New England Biolabs (Beverly, MA). Monoclonal antibodies, anti-DC-SIGN and isotype-matched IgG1 antibody were obtained from Becton Dickinson (Brussels, Belgium). Horseradish peroxydase (HRP)-conjugated goat anti-rabbit or anti-mouse secondary Abs (Amersham, Arlington Heights, IL). The MMP inhibitor, SB-3CT was purchased from Chemicon International (Temecula, CA). Pyridinylimidazole compound, SB 203580, a specific inhibitor of p38 kinase, was purchased from Calbiochem (La Jolla, CA).

### Cell Stimulation

Immature dendritic cells were cultured for 24 hr in serum-free RPMI 1640 conditioned medium. And then infected with DV, cells and supernatant were collected at different periods. For MAPK activation uninfected cells were incubated with 500 ng/mL anisomycin, or 10 ng/ml PMA, as positive control. For inhibition assays, prior exposed to DV, cells were pretreated or not with 10 µM of SB 203580 or SB-3CT for 16 hr.

### Immunoblot Analysis

Infected iDC were lysed in a 1% NP40 buffer. For each time point, equal amounts of protein were electrophoresed under reducing conditions and transferred electrophoretically to nitrocellulose membranes. Membranes were incubated for 30 minutes in Tris buffered saline (50 mM NaCl, 20 mM Tris HCl, pH 7.5) containing 5% bovine serum albumin (BSA) and 0.1% Tween 20 and then incubated overnight with the phosphospecific p38-kinase (T180/Y182) antibody and the anti-active MAPK that recognizes the dually phosphorylated T202/Y204 form of ERK1/2. Proteins were visualized by using the enhanced chemiluminescence system (Amersham Pharmacia Biotech, Piscataway, NJ). Blots were washed in Tris buffered saline containing 0.1% Tween 20 and incubated with horseradish peroxidase-conjugated goat anti-rabbit secondary antibody (Amersham Pharmacia Biotech).

### Detection of Secreted Gelatinase Activity

DV infected iDC (5x105 cells) were cultured and collected in several time periods. Gelatinase secretion of cell supernatants was determined by sodium dodecyl sulfate-polyacrylamide gel electrophoresis (SDS-PAGE) zymography by using a gelatin substrate, as previously described (Missé et al., 2001). Briefly, equal volumes of culture supernatant (500 µL) were lyophilized, resuspended in loading buffer, and, without prior denaturation, were run on 7.5% SDS-polyacrylamide gel containing 1 mg/mL gelatin. After electrophoresis, gels were washed to remove SDS and incubated for 18 hours at 37°C in a renaturing buffer (50 mM Tris, 5 mM CaCl2, 0.02% NaN3, 1% Triton X-100). Gels were subsequently stained with Coomassie brilliant blue G-250 and destained in 30% methanol/10% acetic acid (vol/vol) to detect gelatinase secretion.

### Flow Cytometry Analysis

Immature DC (105 cells) were resuspended in 50 µL phosphate-buffered saline (PBS), supplemented with 3% BSA and 0.02% NaN3 in the presence of the relevant MAbs. After 1 hour of incubation with agitation at 4°C, the cells were washed twice in PBS-0.3% BSA-0.02% NaN3 and resuspended in PBS-3% BSA-0.02% NaN3 in the presence of a 1/50 dilution of fluorescein isothiocyanate-conjugated goat anti-mouse antibody (Caltag, Burlingame, CA). After an additional hour of incubation with agitation at 4°C, cells were washed 3 times as described above, resuspended in PBS, and analyzed by single-color flow cytometry by using a FACSort (Becton Dickinson, San Jose, CA) and Lysis II software. Each datum point was represented by mean of fluorescence intensity of 10 000 gated events.

### Cytokines Detection

Supernatant of DV infected iDC were collected at 1 and 6 hours post infection. MMP-2, MMP-9, MMP-13, TIMP1/2, IL-8, TNF-α and VEGF were measured by ELISA (PerbioScience, Brebières Français).

### Permeability Assay

Permeability of HUVEC monolayers cultured on Transwell filters was assessed using FITC-labelled dextrans. Confluent monolayer of HUVEC were prepared on 24-well transwell polycarbonate membranes (In vitro permeability assay Kit, Chemicon international, Temecula, CA) and incubated for 24 hours with supernatants of Dengue virus-infected iDC at 6 hr post infection. After this period, HUVEC were washed twice with PBS and placed into a chamber containing 500 µl of serum free DMEM medium (Life Technologies, Cergy Pontoise, France). Cells were incubated with 150 µl of the FITC-dextran for 15 minutes at room temperature. The cells were removed and the absorbance of the FITC-dextran in the chamber was measured by spectrofluorometer (GENios-TECAN, Trappes, France) at an excitation wavelength of 485 nm and emission of 530 nm.

### Immunofluorescence

HUVEC monolayers were grown to confluence on 22 mm2 glass cover slips. Cells were exposed to supernatant of iDC-infected DV at 6 hr post infection, for 24 hours. Infected cells were collected at 12 or 24 hr post infection, fixed with 3.7% formaldehyde, permeabilized with 0,05% Triton X-100 and blocked with PBS-2% BSA. Cells were incubated with antibodies against VE-Cadherin (Zymed Laboratories) or PECAM (Zymed Laboratories), washed, and subsequently incubated with an FITC-conjugated goat anti-mouse IgG antibody, Rhodamine-phalloidin (Tebu-Bio, Cedex, France) for actin-containing structures and 4',6-Diamidino-2-phenylindole (DAPI) (Tebu-Bio, Cedex, France) nuclear stain. Coverslips were then washed with 0.1% Tween-20 and PBS. Cells were slide-mounted in Mowiol and visualized under the inverted fluorescence microscope

### Results and Discussion

### Dengue Virus Generates a Productive Infection In Human Immature Dendritic Cells

Dengue virus Dengue 2 primarily targets immature dendritic cells after a bite by an infected mosquito vector (Navarro-Sanchez et al., 2003 EMBO report). Immature DC expressing DC-SIGN receptor (Fig. 2A) was infected with Dengue virus at an MOI of 1 (Fig. 2C). This result shows that Dengue Virus can replicated in iDC expressing DC-SIGN, reaching a peak titer of 106 PFU/ml at 5 days post infection.

### Infected iDC with DV Overexpressed MMP-9 and MMP13

The mechanism by which dengue virus causes DHF/DSS remains unclear. Vascular leakage and hemorrhagic syndrome are the clinical features associated with dengue infection. MMPs are proteolytic enzymes known to be responsible for the integrity of the endothelium (Jackson et al., 1997). We wondered whether circulating mediators present in supernatant of DV-infected iDC were able to exhibit a extracellular matrix protease activity. Therefore, metalloproteinase (MMP) activity of culture supernatant of uninfected and infected iDC was detected at different time periods post infection by gelatin zymography. Figure 3H shows zymogram analysis of gelatinase secretion by iDC at various times after Dengue infection. Although infected cells expressed no significant increase of MMP-2 level than uninfected cells, DV-infected iDC showed in every time collection, strong expression of MMP-9 compared to uninfected cells. The highest different expression observed between infected and uninfected cells was in 1 hr post infection. This experiment also shows that MMP-9 is express even in the supernatant of uninfected iDC. MMP-9 is expressed in a time dependent manner and the maximum level expression of this gelatinase is obtained at 12 hours Pl. To investigate whether MMPs, other than gelatinases are regulated in Dengue infection, we studied the activity of MMP-13 (collagenase-3) in supernatant of DV-infected iDC. Casein Zymography shows that MMP-13 is up-regulated after infection of iDC by DV and is expressed in a time-dependent manner (Fig. 31).

These data were confirmed by quantitative level of MMPs by ELISA in the supernatant of DV-infected iDC. As shown in Fig 3A, B and C, the supernatant of infected cells overexpressed MMP-9 and MMP-13 and in a lesser extend MMP-2 compare to supernatant of uninfected iDC. These observations could be explained by the high level of IL-8 and TNF-αobserved in the supernatant of infected cells (Fig 3D and E). Different studies showed that IL-8 and TNF-α up-regulated gelatinases (Holvoet et al., 2003; Li et al., J Immunol, 2003; Luca et al., 1997; Genersch et al., 2000). It also important to note that DHF/DSS patients have high serum level of IL-8 but not DF patients (Ying-Huey Huang et al., Am J Trop Med Hyg 2000). It is important to note that MMP-13 is known to play a central role in the MMP activation cascade, particularly by activating MMP-9 (Leeman et al., J Clin Pathol 2002; Knäuper et al., Eur J Biochem (248) 1997).

### Dengue Virus-Induced Secretion of MMP9 by iDC Involves Activation of the MAPK Pathway

The secretion of certain MMPs, such as MMP-9, has been shown to involve on the MAPK signalling pathway, via the activation of Raf, MEK1/2, and ERK 1/2. In order to investigate whether the DV-induced secretion of MMP by dendritic cells was associated with an activation of the MAPK pathway, we determined the phosphorylation of ERK and p38 in these cells (Genersh et al Journal of Cell Science 2000; Holvoet et al., 2003 Experimental cell research 290, 2003). Infection of iDC by DV resulted in a significant increase in the phosphorylation of both ERK (Fig. 5A) and p38 (Fig. 5B) MAPK. Moreover, the increase in MMP-9 secretion by DV-infected iDC was totally abolished in the presence of 10µM SB203580, a specific inhibitor of p38 MAPK (Fig. 5C), indicating that p38-mediated signaling is essential for the up-regulation of MMP-9, as a result of the interaction of DV with the DC-Sign receptor.

### Induction of Endothelial Permeability Correlated with Release of MMPs from Dengue-Infected iDC

We wondered whether the supernatant of DV-infected iDC was able to change the membrane permeability of endothelial cells. Primary HUVEC monolayer was exposed for 12 hr to supernatants from uninfected or infected iDC and their permeability was assessed (Fig. 6A). Compare to supernatant from uninfected iDC, the supernatant from infected iDC significantly increase permeability in endothelial cells. This assay was validated using recombinant TNF-α (as positive control), a known inducer of endothelial permeability. To assess if production of permeabilizing activity related to gelatinases secretion after infection of iDC with DV, HUVEC were incubated in the presence or absence of a specific inhibitor of gelatinases, SB-3CT of gelatinases, SB-3CT. Figure 6A shows that the increase of the endothelial permeability in DV-infected iDC is totally inhibited with this inhibitor, the permeability decrease to a basal level corresponding to the incubation of HUVEC with iDC supernatant. Interestingly, recombinant TNF-α increase endothelial permeability can also be strongly inhibited by SB-3CT. Figure 6B shows a dose response inhibition of HUVEC permeability by SB-3CT. The infection of iDC by DV and the subsequent production of MMP have clearly deleterious effects on the integrity of the endothelium and may contribute to vascular instability, explaining the haemostatic impairments noted in DHF/DSS. Dendritic cells are migratory cells which exhibit complex trafficking properties in vivo, involving interaction with vascular and lymphatic endothelium and extracellular matrix. Endothelial porosity may also facilitate endothelial transmigration of infected DC. This observation is consistent with the findings of others investigators, in particular its has been demonstrated that MMPs are considered to be essential for migration of NK cells, Langherans cells and T cells (Leppert, J Immunol 1995, Kitson, J Immunol 1998, Kobayashi, J Immunol 1999). DC infected with DV used probably the MMP for invasion to the secondary lymphatic organs to stimulate the specific T-cell.

### Dengue Virus-Infected iDC Disrupt Endothelial Cell-Cell Adhesion by an MMP-Dependent Mechanism

Endothelial cells express cadherins and PECAM-1 and contain an extensive F-actin cytoskeleton, which are implicated in changes of cell shape and junctional assembly/disassembly (Hordijk et al., 1999, Journal of Cell Science 112, 1915). In order to visualize whether DV-infected iDC were able to modified the integrity of the endothelium, we analyzed the distribution of PECAM-1 and VE-cadherin as well as the content of F-actin in confluent monolayers of HUVEC, incubated in the presence or absence of culture supernatant of infected iDC (Fig. 8). Immunostaining showed that cell-cell adhesion of HUVEC monolayer was significantly reduced in the presence of iDC-DV supernatant (Fig 8B and 8E). In addition, MMP activity on cell integrity in the HUVEC monolayer was reflected by a decrease of F-actin staining, indicating a reduction in the actin stress fiber content (Fig 8H). To investigate whether the observed disruption of cell-cell adhesion induced by the culture supernatants of infected iDC was MMP-dependent, we repeated the experiment in the presence of the specific MMP inhibitor SB-3CT. As shown in figure 8C and 8F, the loss of junctional localization induced by MMP contained in these supernatants was blocked by this inhibitor. The inhibition of MMP function protects the proper organization of the endothelial actin cytoskeleton and maintains cell-cell adhesion (Fig 81). The cytopathic effects induced by MMP correlated with broad modifications of the microfilament network and could be blocked by the MMP inhibitor SB-3CT.

## Claims

1. A method of treating viral haemorrhagic fever, which comprises administering a composition comprising a pharmacologically active amount of a matrix metalloproteinase (MMP) inhibitor to a patient in need thereof.

2. A method according to claim 1, wherein the haemorrhagic fever is caused by a virus of the family Arenaviridae, Bunyaviridae, Flaviviridae or Filoviridae.

3. A method according to claim 2, wherein the virus is Dengue virus.

4. A method according to any of claims 1 to 3, wherein one or more of MMP-2, MMP-9 and MMP-13 is inhibited.

5. A method according to any preceding claim, wherein the MMP inhibitor is a specific MMP inhibitor.

6. A method according to claim 5, wherein the inhibitor is SB3CT or a derivative thereof.

7. A method according to any of claims 1 to 4, wherein the inhibitor interferes with the MAPK signalling pathway.

8. A method according to claim 7, wherein the inhibitor is an inhibitor of MAPK p38.

9. Use of an inhibitor of matrix metalloproteinase (MMP) in the manufacture of a medicament for the treatment of viral haemorrhagic fever.

10. Use according to claim 9, wherein the haemorrhagic fever is caused by a virus of the family Arenaviridae, Bunyaviridae, Flaviviridae or Filoviridae.

11. Use according to claim 10, wherein the virus is Dengue virus.

12. Use according to any of claims 9 to 11, wherein one or more of MMP-2, MMP-9 and MMP-13 is inhibited.

13. Use according to any of claims 9 to 12, wherein the MMP inhibitor is a specific MMP inhibitor.

14. Use according to claim 13, wherein the inhibitor is SB3CT or a derivative thereof.

15. Use according to any of claims 9 to 12, wherein the inhibitor interferes with the MAPK signalling pathway.

16. Use according to claim 15, wherein the inhibitor is an inhibitor of MAPK p38.
